# EUROPEAN PATENT SPECIFICATION

(11) **EP 1 446 168 B1**
(45) Date of publication and mention of the grant of the patent: **15.08.2007**
(21) Application number: 02779707.5
(22) Date of filing: 20.11.2002
(51) Int. Cl.: A61L 15/60, A61L 15/48, A61L 15/26, A61L 26/00

(54) **MEDICAL DRESSINGS**
MEDIZINISCER VERBAND
PANSEMENTS MEDICAUX

(30) Priority: 20.11.2001 GB 0127822
(43) Date of publication of application: 18.08.2004
(73) Proprietor: Maelor Pharmaceuticals Limited, Newbridge, Wrexham LL14 3JG (GB)
(72) Inventor: Rippon, Mark, Geoffrey Maelor Pharmaceutical Ltd., Newbridge Wrexham LL14 3JG (GB); Meadows, John Maelor Pharmaceutical Limited, Newbridge Wrexham LL14 3JG (GB)
(74) Representative: Lord, Hilton David
(86) International application number: PCT/GB2002/005221
(87) International publication number: WO 2003/045452

(56) References cited:
- EP-A- 0 386 960
- EP-A- 0 470 007
- EP-A- 0 481 600
- WO-A-97/00275
- GB-A- 2 124 488
- GB-A- 2 362 100
- US-A- 4 920 158
- US-A- 5 718 916
- US-A- 5 840 338

## Description

The present invention relates to dressings for exudative wounds, and other medical dressings.

Skin damage often leads to haemorrhage and blood coagulation to form a clot, and mediators released by clot formation stimulate cells involved in healing. Primarily, inflammation occurs, during which immune cells infiltrate the wound, epithelial cells migrate and proliferate, and fibroblasts undergo phenotypic changes. Overlapping with this phase is the granulation stage, which involves formation of neovasculature by endothelial cells and synthesis of immature collagen and specific extracellular proteins by fibroblasts. Finally, during matrix formation and remodelling, mature collagen is cross-linked, different extracellular matrix proteins are deposited, and tissue matrix is formed into scar tissue, with reduced vascularity

Wounds can be classified according to the amount of damage caused to the skin. For example, partial-thickness wounds penetrate the outer layers of the skin, the epidermis and the superficial dermis, and heal by regeneration of epithelial tissue. Full-thickness wounds involve a loss of dermis and may include deep tissue, as well as disruption of the blood vessels. These wounds generally heal by secondary intention, whereby the lost tissue is regenerated by appropriate cell types and results in the production of scar tissue.

Wounds can further be divided into either acute or chronic. Generally, acute wounds follow roughly the same healing process. They heal by minimal intervention, other than the application of a simple dressing that protects the wound from further trauma and prevents infection from external sources. Chronic wounds, on the other hand, are difficult to heal and may last many years. Their aetiology is complex and related to many factors, including underlying pathology, environment, nutrition and treatment regimes. Examples of chronic wounds are leg ulcers, including arterial, venous and diabetic ulcers, pressure sores, malignant wounds and burns.

One of the most important decisions in wound management is the choice of dressing. The user has a wide variety of dressings to choose from, and selection is often governed by tradition, habit or financial considerations, rather than by scientific knowledge. Traditional wound dressings have comprised a fabric or a felt of absorptive material, such as gauze, in direct contact with the wound. Such dressings fulfil a number of basic functions, such as cosmesis, haemostasis, protection, support and absorption.

However, these dressings have a number of drawbacks. They stick to the wound and, thus, interfere with the process of healing, and may also dry the wound out and adhere to the surface of the wound, causing re-traumatisation upon removal.

Current medical thinking has wounds as temporary organs, which are established by the body to effect healing. Thus, dressings may be designed to nurture the cellular environment of the wound. They may also be designed to debride the wound, control infection and promote healing, thereby facilitating the function of the wound organ, rather than just covering it. Accordingly, currently accepted dressing types can be characterised as follows:

| | |
|---|---|
| Superficial partial thickness | Adhesive film |
| Mild to moderate exudate | Hydrocolloid/hydrogel |
| Contaminated, moderate to heavy exudate | Alginate |
| Heavy exudate | Foam |
| Dry necrotic | Amorphous Hydrogel |

Wound dressings are now often either designed to enhance autolytic debridement, or to contain lytic or other agents, such as collagenase, that actively enhance the debridement process.

The wound environment, therefore, is important in determining how well and how quickly, and even whether, the lesion will heal, the most favourable environment being a moist wound.

Chronic wounds, such as leg ulcers, burns and pressure sores, exude large amounts of wound fluid. This aspect of chronic wounds provides a significant problem to the majority of traditional wound dressings, such as gauzes, in that the limited level of exudate that they can manage ultimately results in exudate leakage and accumulation beneath the dressing. The exudate may contain pathogenic bacteria which, if left in the wound environment, may cause at first localised and subsequently systemic infection, without adequate control. The wound exudate from a chronic wound also contains large amounts of proteases (e.g. metalloproteases, plasmin, elastase and hyaluronidase), which have been demonstrated to significantly impair the repair process by breaking down the matrix components of the neo-granulation tissue, thereby preventing cellular proliferation, migration and matrix deposition. Leakage of exudate onto surrounding healthy tissue can also result in tissue maceration and exacerbation of the wound.

Highly absorbent dressings are now available, and these have been designed to prevent pooling and contact of exudates with the fragile surface of the wound and surrounding skin. For example, the hydrocolloids are used for shallow ulcers, and are generally applied directly over the wound. They form a gel interface and are impermeable to oxygen, moisture and bacteria. Their hydrocolloid properties maintain a moist wound surface, support autolytic debridement and provide some degree of exudate absorption. The gel interface does not adhere to the wound surface and, so, does not disrupt granulation tissue on removal.

US-A-5762620 discloses a wound dressing containing a partially de-hydrated hydrogel gauze wound dressing, designed to absorb large amounts of wound exudate without inhibiting the wound through direct contact. Additionally, Mason, Jr., *et al.*, in US-A-4,393,048, disclose a dry powdered wound treatment composition which, following introduction into an open, draining wound, forms a hydrogel capable of absorbing wound exudate. However, dry hydrogel deteriorates as the wound fluids are absorbed, resulting in lumping and uneven application. These lumps are difficult to remove from a wound site when the dressings need to be changed, and the removal process may cause damage to the fragile new tissue developing.

In wounds that demonstrate very high levels of wound exudates (e.g. venous leg ulcers and pressure sores) then preferred dressings include the alginates, which are derived from seaweed. Alginates provide highly absorbent, biodegradable dressings, and are disclosed by Kershaw in US-A-5,986,164, for example. The high absorption is achieved as a result of strong hydrophilic gel formation.

Alginate dressings maintain a physiologically moist microenvironment that promotes healing and the formation of granulation tissue. Generally, when fully hydrated, alginates can be rinsed away by saline irrigation, so that removal of the dressing does not interfere with the healing granulation tissue. With some alginate dressings, fibres may become trapped and cause irritation to the wound. In addition, some alginate dressings (e.g. Algosteril) demonstrate significantly greater adherence to the wound surface than other similar alginate dressings, resulting in greater tissue damage upon removal. Although any remaining fibres will eventually dissolve, their continued presence can result in an undesirable immune response.

GB-A-1,440,191 discloses a pervious surgical adhesive dressing which has an absorbent pad comprising a resilient foamed plastics material and a facing layer of a fibrous non woven fabric. The resilient foamed plastics materials disclosed in this patent are not hydrophilic per *se* and need treatment with surfactants to give them adequate absorption properties.

Foams are used as an adjunct for heavily exudative wounds. Foam and foam film dressings are available in sheet form and as cavity dressings. Foam dressings generally provide thermal insulation, maintain a moist wound environment and are comfortable to wear. Foam dressings are used in a variety of wounds; including leg ulcers and pressure sores, and are suitable for light, moderate or heavily exuding wounds, depending on the product. As most foam dressings rely on exudate to achieve an optimum healing environment, they are not suitable for dry epithelialising wounds or dry eschars.

Although such wound dressings and surgical sponges have been found useful in the past for absorbing large amounts of wound exudate, nearly all of these dressings, including gauze and sponges, adhere to the wound upon removal, thereby damaging the wounds to which they are attached.

WO 01/85845 discloses hydrogels comprising poloxamers, the poloxamers providing the advantage of increased viscosity and adherence to the wound.

WO 00/41732 teaches the use of hydrogels for delivery of viral vectors. These dressings can be stiffened with collagen fibres and may include a component selected from the group consisting of a poloxamer and an alginate.

WO 98/48768, is concerned with reversible gelation for cosmetic patches and discloses a poloxamer: poly(acrylic acid) polymer network, the poloxamer forming part of the polymer network of the gel, and the gel aggregating in response to an increase in temperature.

WO 98/42348 discloses a lecithin organogel in combination with a poloxamer, used as a dressing or other topical application, prevents drying and subsequent cracking of the skin.

Accordingly, there is a need for a wound dressing capable of absorbing large amounts of wound exudate, but with reduced adherence to the wound surface.

Surprisingly, it has now been found that application of a poloxamer to a medical dressing significantly reduces wound adhesion.

Thus, in a first aspect, the present invention provides a dressing for an exudative wound, wherein at least that part of the dressing intended for contact with the wound has been coated with at least one poloxamer.

The term "coated" is explained in more detail, below, and is used herein to indicate that the poloxamer is applied to the dressing, either in a continuous or discontinuous fashion. The effect is to provide a coating to the fibres or other material of the dressing, rendering the coated portion of the dressing less likely to adhere to the wound after the dressing has been left in place for a period of time, such as a dressing might normally be left in place for.

The coating of poloxamer may be achieved as described below. In general, it is sufficient that the poloxamer reduces the tendency of the dressing to adhere to the wound after having been left in place for a period of time. Without being restricted by theory, it is likely that the poloxamer coats the fibre or material of the dressing to provide a layer of surface active agent to reduce adhesion. An amount of poloxamer, generally a majority, is likely to penetrate the fibre or other material of the dressing, especially where only one coat is applied, and this is acceptable, provided that the tendency of the dressing to adhere to a wound, after a period of time, is reduced.

It will also be appreciated that the level of poloxamer required to reduce adhesion may only be relatively small, and is discussed in more detail below. Once a level has been reached that effectively coats the entire surface area of the dressing in contact with the wound, then any additional poloxamer may not be necessary for this purpose, but may serve to carry drug, for example.

The poloxamer may be applied in one or more layers, typically allowing drying between applications, if a diluent or carrier is used. Where it is desired to provide a layer of poloxamer on the dressing, then it is preferred to apply at least two coatings of poloxamer.

In addition, multiple poloxamers may be employed, either together or applied separately. This is particularly advantageous where it is desired to control release properties of any drugs or other materials carried by the dressing.

In an alternative aspect, the present invention provides a dressing for an exudative wound, wherein that part of the dressing intended for contact with the wound has been treated with a poloxamer.

The present invention relates to any dressing that may be applied to any wound that is exudative. Essentially, the poloxamer of the dressing prevents, or inhibits, the dressing from adhering to the wound through any stickiness, moisture or fluid associated therewith. Thus, if the wound is dry, then a dressing of the present invention may not be necessary, although this does not prevent such a dressing being used if desired.

In general, it is preferred that dressings of the present invention are absorptive, and even standard gauze dressings may be advantageously treated with poloxamers to minimise adhesion on removal.

It will be appreciated that minimising adhesion on removal has the advantage of also minimising the amount of trauma associated with change of wound dressing. This, in turn, has the advantage of minimising pain, which has been demonstrated to hinder wound healing, and also has the advantage of minimising any disruption to any on-going epithelialisation.

More preferably, dressings of the present invention are highly absorbent, and may be selected from foams, foam films, and alginates, as well as other highly absorbent dressings. Alginates, and especially fibrous alginates, are preferred.

In the case of foams and foam films, it will be appreciated that generally only that part of the foam or film that will form part of the interface with the wound needs to be treated with the poloxamer. Thus, a simple spray of the surface of the finished dressing may suffice to provide the dressing of the present invention.

It will also be appreciated that any poloxamer used in the present invention may have an effect on the amount of fluid that can be absorbed by the dressing. Where the amount of poloxamer is small, it may even serve to enhance the amount of fluid that can be absorbed. However, where the amount of poloxamer is large, then this may displace the amount of exudate that can be absorbed by the dressing. Accordingly, it is generally preferred to minimise the amount of poloxamer used in dressings of the present invention, whilst ensuring that as much as possible of the surface of the dressing that is intended to come into to contact with the wound is covered.

Similar considerations apply to fibrous dressings, such as the alginates. In general, it is preferred that dressings of the present invention are fibrous dressings, especially fibrous alginate dressings. Again, it is generally preferred to ensure that the interface is treated with poloxamer. In some instances, it may also be desirable to ensure that the poloxamer permeates into the body of the dressing, as the main interface tends to form a gel, and some of the fibres located within the dressing may come to interact with the exudate of the wound.

In general, dressings of the present invention will have backing layers, and these are preferred to be occlusive to the passage of water and other liquids, although it is often preferred that they allow the wound to breathe. Such backings will be permeable to water vapour and oxygen, but not to water and other fluids.

It is also generally preferred that the backing have an adhesive area suitable to adhere in the vicinity of the wound, on healthy skin, but this is not necessary, and it may be sufficient simply to provide a wadding of dressing to an area, and then securing this wadding with a bandage or other material, such as described above in relation to the backing.

Suitable examples of dressing without backing include ribbons and ropes of alginates which may be wadded into open wounds. Other examples will be readily apparent to those skilled in the art.

It will be appreciated that the poloxamer should cover at least a part of the dressing that is intended to come into contact with the wound. Preferably, the poloxamer should be used to treat all of that part of the dressing which may come into contact with the wound. In this respect, it will not be expected to cover the, or any, adhesive used to secure any backing to the periphery of the wound, as it is not generally intended for this to come into contact with the wound. Essentially, it is generally preferred for the poloxamer to be used to treat any exposed surfaces of the absorptive material.

Poloxamers vary greatly in their constituent make up, and are generally characterised by the ratio of ethylene oxide units to propylene oxide units, and the molecular weight of the propylene oxide block. Poloxamers comprise PPO units and EO units. The PPO units are relatively hydrophobic, and form the central portion of any micelle-type structures which can occur in aqueous solutions of poloxamers, depending on temperature and concentration.

Individually preferred poloxamers are P188, P234, P237, P331, P338 and P407. P331 is a relatively poorly water soluble poloxamer which is used in pharmaceutical tablets, for example, and can be useful in controlling or affecting release rates of actives from the coated dressings of the present invention.

Poloxamers have previously been shown to have anti-adhesion properties. For example, it has been demonstrated that bacterial adherence of staphylococci to polymethylmethacrylate (an acrylate cement used in orthopaedic surgery) was markedly inhibited (77-99%) when the substrate was exposed to (P407) before or during an adherence assay. The data also indicated that the poloxamer was interfering with bacterial adherence and biofilm formation [Veyries et al., Antimicrob. Agents Chemother. 2000 Apr; 44(4):1093-6]. Portoles et al. [Cornea. 1995 Jan; 14(1):56-61] have also described the abherent (anti-adherent) effect of P407 on *Pseudomonas aeruginosa* to corneal epithelial cells. Poloxamer 407 significantly inhibited 92-99% *of Pseudomonas aeruginosa* adherence to hydrophilic contact lenses. This adherence inhibition was concentration- dependent. A reduction of about 50-60% was obtained for Staphylococcus strains, and 50-70% for Gram-negative strains other than Pseudomonas. However, in this study the authors indicated that the poloxamer seemed to prevent bacterial adhesion by acting on the surface of the bacteria and not on the contact lens surface.

In addition, poloxamers have also been shown to have anti-bacterial effects, but the mechanism of the anti-bacterial effects is not known. However, it has been shown that surfactants interfere with or bind to the surface of and or components of bacterial cell membranes. For example:- in 1978, Zaki & Abdel-Samie demonstrated that non-ionic surfactants affect the integrity of the cytoplasmic membrane; cellular lysis of *Streptococcus faecalis* was demonstrated to be induced by both anionic and non-ionic surfactants (Cornett & Shockman, 1978); Nod & Kanemasa demonstrated that non-ionic surfactants were adsorbed to the hydrophobic sites of bacterial cell surfaces; and it has been shown that mixed surfactants bind with bacterial lipopolysaccharide molecules (Panda & Chakrabarty, 1998); more recently it has been shown that cell membrane damage morphology change and rupture of bacteria by ionic surfactants has been demonstrated and shown to be caused by damage to the lipid bi-layers of the bacterial membranes (Groot & Rabone, 2001).

It does not appear, however, that the membrane damaging effects caused by poloxamers to bacteria are reproduced in cells of mammalian origin. On the contrary a number of workers have shown that damaged cells treated with poloxamers can retain their former integrity, and that poloxamers can be used to repair damaged cells/cell membranes.

Thus, the use of poloxamers in the present invention may also confer useful anti-bacterial and tissue-protective properties on the dressing.

Surprisingly, it has now been found that it is possible to usefully incorporate other substances into the dressing together with the poloxamer. There is no particular limit on the substance that may be incorporated into the poloxamer, provided that it is capable of being solubilised either by the poloxamer alone, or in the presence of the poloxamer and one or more co-solvents, such as water, acetone and/or polyethylene glycol. It will be appreciated that such additional substances need not necessarily be incorporated into the dressing together with the poloxamer, and may be used to treat the dressing separately, if desired, preferably as an appropriate solution.

Poloxamers are surfactants, and surfactants are amphiphilic substances. In other words, they comprise both hydrophilic and hydrophobic regions, and are commonly used to solubilise fatty substances in water. Above certain concentrations in water, surfactants tend to form micelles - agglomerations of surfactant molecules presenting their hydrophilic portions to water and internalising the hydrophobic portions. With increasing concentration, other structures may also be observed, but these tend to be somewhat complex. In the obverse, each surfactant has a minimum concentration in water below which micelles disperse (critical micelle concentration - CMC), and the aqueous surfactant preparation is effectively a solution of unimers with no structure.

Surfactant micelles are effectively envelopes and, in water, will have the more hydrophobic portion of the molecule generally forming the inside of the envelope. In the present invention, it is likely that any hydrophobic substances incorporated into the dressing are incorporated via poloxamer micelles where the poloxamer is present as an aqueous solution, or an aqueous solution with a further co-solvent, such as polyethylene glycol. Thus, these micelles can readily interact with other substances and, if the substance is an oil, for example, then the substance can be entirely internalised within the micelle, or otherwise form an association, thereby effectively solubilising the substance in water.

The nature of the poloxamer is not essential to the present invention although, especially where the formulation is intended for administration to a human, it should be pharmaceutically acceptable.

Poloxamers are generally unreactive and non-responsive to any other additives to the system, such as BSA (Bovine Serum Albumin) or salt, such as sodium chloride. In addition, pH appears to have little, or no, effect. Thus, there is no problem with incorporating other suitable substances into the poloxamer which it may be desired to incorporate into the dressing, optionally for dispensing to the wound.

It will be appreciated that the dressings of the present invention may incorporate more than one poloxamer, and it is now well established that combinations of poloxamers may synergistically dissolve various substances, such as the anaesthetic, Propofol. In general, it is not preferred to incorporate Propofol into dressings of the present invention, as this is a general anaesthetic. However, it is a particular advantage of the present invention to incorporate a local anaesthetic, such as lidocaine or Prilocaine and, indeed, it has been demonstrated that the presence of lidocaine is sufficient to reduce the cellular adherence properties of alginate, for example, still further.

Pain is prevalent in the majority of both acute and chronic wounds, precluding those of neuropathic origin or those which have associated nerve damage. Pain is particularly prevalent in wounds of a chronic disposition such as bums, pressure sores and leg ulcers. Associated wound pain is detrimental to the overall health of the patient, and has been shown to have negative effects on the rate at which a wound will heal.

Debridement of the recalcitrant wound is a recommended practice required in order to remove dead necrotic and sloughy tissue, and this treatment is necessary to allow healing to proceed. The practice of mechanical debridement involves the use of a sharp curette or scissors to remove the non-viable tissue. This process is extremely painful to the patient both during and after such treatment. It has been shown that patients undergoing sharp debridement without anaesthesia experience post-debridement pain for up to four hours afterwards. The major currently marketed product that has been used in these types of treatment modalities is EMLA cream, which is a eutectic mixture of lidocaine 2.5% and prilocaine 2.5%. A large number of studies has shown this treatment to be effective in the treatment of pain associated with the debridement of these types of wound, with low systemic lidocaine levels and no adverse effect on healing.

Thus, the dressings of the present invention preferably further comprise a local anaesthetic, preferably lidocaine or prilocaine.

Other substances that may be incorporated in the dressings of the present invention include antibacterials, antifungals, anti-inflammatory agents, anti-cancer agents and conventional wound treatment agents, including any suitable form of debridement and growth factors, as well as other agents suitable to encourage wound healing.

It will also be appreciated that any combination of the above may suitably be used in dressings of the present invention, especially a combination of a local anaesthetic with one or more other substances to encourage wound healing.

Application of poloxamer to dressings of the present invention may take place in any suitable manner, including immersing the dressing in poloxamer, or a preparation of poloxamer. In general, for the reasons given above, it is preferred not to soak the dressing in a preparation of poloxamer. Apart from anything else, this may cause the dressing to prematurely gel, should the dressing be of the gelling variety.

More generally, it is preferred to drizzle, spray or mist the poloxamer onto the dressing, or any other suitable method, short of soaking by immersion.

Where the poloxamer is sufficiently mobile, then it may be possible to spray this directly onto the dressing. Otherwise, it is preferred to prepare the poloxamer, or poloxamers, as a solution, either in water, or in an organic co-solvent, or mixture thereof. In either case, once the dressing has been treated with the preparation of the poloxamer, then it is preferred to remove the solvent, such as water or acetone, after treatment. This can either be by heating, or simply by maintaining the dressing at ambient temperature in suitable dry conditions.

Where one or more additional substances are to be incorporated into the dressing, then it is preferred to incorporate such additional substances into the poloxamer, or solution of poloxamer, prior to treating the dressing.

It is also possible to employ more than one of the above methods to treat a dressing of the present invention. Thus, it is possible to treat one side of the absorbent part of the dressing with a poloxamer prepared with an organic co-solvent and a substance to be dispensed, such as lidocaine, and the other side of the dressing with an aqueous preparation of poloxamer containing the same, or a different, substance. In this case, it is anticipated that the side of the dressing treated with the aqueous suspension will initially come into contact with the wound, and rapidly dispense the substance contained therein, whilst the substance contained in that part of the dressing prepared with the organic preparation will permeate more slowly, owing to both the method of treatment and the lack of proximity to the wound interface. This latter side may be in close proximity, or be secured to, any backing layer.

Alternatively, for example, the methods may be used to layer poloxamer and any additional substance onto the dressing, typically drying, or substantially drying, the dressing between applications, thereby achieving a similar effect.

The effect can be substantially enhanced by the choice of poloxamer. For example, a highly water soluble poloxamer and a less water soluble poloxamer can be used. Highly water soluble poloxamers are P234 and P407, for example, while less readily water soluble poloxamers are, for example, P331 and P401. In this case, P331 or P401 can be prepared in acetone and lidocaine incorporated therein, and the solution used to layer into the dressing. The acetone can then be removed by evaporation, such as under reduced pressure at ambient temperature. A subsequent aqueous preparation of P234 and lidocaine, optionally containing polyethylene glycol, for example, can then be layered on, or in, and dried, such as at a raised temperature between 80°C and 90°C, for example. The resulting dressing can then rapidly dispense lidocaine on initial application and via the readily soluble P234 or P407, and also deliver the lidocaine associated with the P331 or P401 in a sustained manner, owing to the reduced solubility of the preparation.

Suitable amounts of poloxamers are as described, but it has been found that the more highly water soluble poloxamers, such as P407 and P234, may usefully be applied in amounts of between 5 and 15%, such as 8 to 12% w/v, while less soluble poloxamers, such as P331 and P401, may usefully be applied in amounts of between 5 and 80%, such as 20 to 50% w/v.

Alginates are highly absorbent materials derived from seaweed, primarily comprising guluronic acid and mannuronic acid. High guluronate content gives strength and the dressing will gel slowly, while dressings with a high mannuronate content are more soluble and will gel more quickly. Alginate dressings absorb large quantities of fluid, giving longer wear time compared with conventional gauze dressings.

For shallow, heavily exuding wounds such as leg ulcers, fibrous sheet dressings made from alginate fibre may be used, while cavity wounds, traditionally packed with gauze soaked in saline, hypochlorite, or proflavine, are now more commonly dressed with alginate fibre in the form of ribbon or rope. For epithelialising wounds, alginates have an advantage over cellulose dressings, in that they can more easily be removed if they are first well soaked with sodium chloride solution.

Calcium alginate is insoluble in water but, in the presence of sodium ions from wound exudate, a partial ion exchange reaction takes place resulting in the production of sodium alginate which forms a hydrophilic gel on the wound surface that is believed to facilitate healing.

In the case of alginates, the treatment with poloxamers is also useful in that there is a reduced occurrence of the shedding of fibres by the dressing on removal. As noted above, this is advantageous, as fibres remaining in the wound can lead to an immune response.

Dressings of the present invention preferably comprise fibrous alginate treated with a poloxamer and a local anaesthetic, especially lidocaine. Such dressings are highly absorptive, easy to remove from delicate wounds and have immediate anaesthetic qualities to minimise any trauma the wound may have suffered.

The amount of poloxamer needed to treat dressings of the present invention is, as noted above, whatever is sufficient to cover an area of the dressing that is to come into contact with the wound. Where this is the only requirement for the poloxamer, then levels as low as 0.5% by weight of the absorptive part of the dressing may be used. Levels as high as 150% or even 200% of the weight of the dressing may also be used, and still provide for water or exudate absorption. However, it is preferred that the amount of poloxamer should not exceed 100% by weight, and it is also preferred that the minimum be between 1% and 10% by weight, with an upper limit of about 50% by weight where no extra substance is being incorporated with the poloxamer.

Where a substance such as lidocaine is being incorporated into the poloxamer, it will be appreciated that the amount of the substance will be dictated by the effect it is intended to have in the dressing. Some substances have effects in minuscule quantities, such as growth enhancers and hormones. Suitable quantities of these will be readily apparent to those skilled in the art. Likewise, quantities of substances such as lidocaine may vary between about 0.5% and 10% by weight of the poloxamer.

In the following Examples, the Figures are as follows:
Figures 1 to 4 illustrate the release of three model dyes using varying amounts, layers and mixtures of P331 and P407;
Figures 5 and 6 illustrate the results of cell release studies using granulation tissue cells;
Figures 7 and 8 illustrate the results of cell release studies using L929 cells; and
Figures 9 and 10 illustrate the effect of the presence of a surface layer of P331 on the adherence of various cell types to fibrous alginate dressings.

The present invention will be further illustrated by the following, non-limiting Examples. In these Examples, all percentages are by weight, unless otherwise indicated.

### EXAMPLE 1

### Coated Alginate Dressings

### Materials

Poloxamer 234 (Lutrol L-84; BASF)
Lidocaine (Sigma)
Fibrous alginate dressings (10×10 cm; Maersk medical; 1.1 g dry weight)

### Stock solutions

20 cm³ of each of the following was prepared;
(i) An aqueous solution containing 2 % w/v P 234 and 0.2 % w/v lidocaine in distilled water.
   This was prepared by dissolving 4g of poloxamer 234 in 20 cm³ of distilled water and then adding 0.4g of lidocaine. The resultant mixture was tumble mixed for a period of 24 hours to allow complete solubilisation of the lidocaine to occur.
(ii) A non-aqueous solution comprising 2 % w/v P 234 and 0.2 % w/v lidocaine in acetone. This was prepared by dissolving 4g of poloxamer 234 in 20 cm³ and 0.4g of lidocaine in 20cm³ of acetone

### Coating of dressings

The coating protocol was designed to produce dressings coated with approximately 1 % w/w lidocaine and 10% w/w poloxamer 234 with respect to the weight of the dry dressing.

### Aqueous route

An alginate dressing was accurately weighed and placed in an appropriately sized petri dish. Using a small graduated syringe, 6 cm³ of the aqueous P234/lidocaine solution was added to the alginate dressing by means of evenly spaced drops, wetting as much of the surface of the dressing as possible during the process. The petri dish and dressing were placed in an oven and dried at 60°C for two hours, after which the dish and dressing were allowed to cool to room temperature.

### Acetone route

An alginate dressing was accurately weighed and placed in an appropriately sized clock glass. Using a small graduated syringe, 6 cm³ of the acetone based solution P234/lidocaine was carefully added to the alginate dressing by means of evenly spaced drops, wetting as much of the surface of the dressing as possible during the process. The clock glass dressing was placed in a fume cupboard and allowed to dry at ambient temperature. After the drying process was complete, the dressing was carefully transferred to an appropriately sized petri dish.

### EXAMPLE 2

### Preparation of a lidocaine containing dressing

### Method

5 cm³ of a 5% w/v aqueous solution of lidocaine hydrochloride also containing 4 % w/v Poloxamer 407 was slowly drizzled onto a 10 cm x 10 cm fibrous alginate dressing (Sorbsan; ex- Maersk Medical) placed within a glass dish. The dish and dressing were then placed in an oven and dried at 60°C for two hours.

### EXAMPLE 3

### Preparation of a metronidazole containing dressing

### Method

5 cm³ of a 2% w/v aqueous solution of metronidazole, also containing 2 % w/v Poloxamer 407, was slowly drizzled onto a 10 cm x 10 cm fibrous alginate dressing (Sorbsan; Maersk Medical) placed within a glass dish. The dish and dressing were then placed in an oven and dried at 60°C for two hours. The dressing was removed, allowed to cool and then 5 cm³ of an 8 % w/v solution of Poloxamer 331 in acetone was drizzled onto the dressing. The dressing was then left to dry under ambient conditions.

### EXAMPLE 4

### Active Release Studies

When measuring the release of actives such as lidocaine or metronidazole from the coated dressings using UV absorbance as the analytical method, interference problems can arise, owing to the release of entrained surfactant material from the dressing itself. In the case of alginate fibres, it is thought that the surfactant type material is an anti-twist additive used in the finishing of the alginate fibres, and has a UV absorbance overlapping those of the actives under study. Thus, it was decided to study the release rates of a series of dyes, whose UV absorption bands were at considerably higher wavelengths than the surfactant impurity and which also offered the advantage of providing a ready visual evaluation of the active release. The three dyes chosen were water soluble anionic, water soluble cationic and water insoluble non-ionic, thereby corresponding to a range of characteristics of actives.

### Method

The effect of poloxamer type and surface layer composition on the release of active materials was studied in the following manner. A series of coated dressings (10 x 10 cm) were prepared as described in Example 1, with surface coatings comprising P407, P331 or both. The surface coatings also contained various amounts of one of three model dye compounds, namely (i) water soluble, anionic Acid Red 18, (ii) water soluble cationic methylene blue or (iii) the poorly water soluble, non-ionic methyl violet.

The rates of release of the various model compounds were determined by supporting the dressings on a wire mesh, such that the coated surface of the dressing was just in contact with the surface of a known volume (500 cm³) of distilled water contained within a glass beaker. The bulk solution was gently stirred throughout to enable homogeneity of the bulk solution to be attained prior to the periodic sampling of the bulk solution over a period of several hours. After each small sample of the bulk solution had been taken, an equivalent volume of distilled water was added to the bulk in order to maintain a constant volume and, hence, not interfere with the positioning of the dressing at the air-water interface. The concentration of released dye in the various samples was determined by UV absorbance spectrophotometry, enabling the amount and rates of dye released from the various dressings to be monitored over the duration of the study.

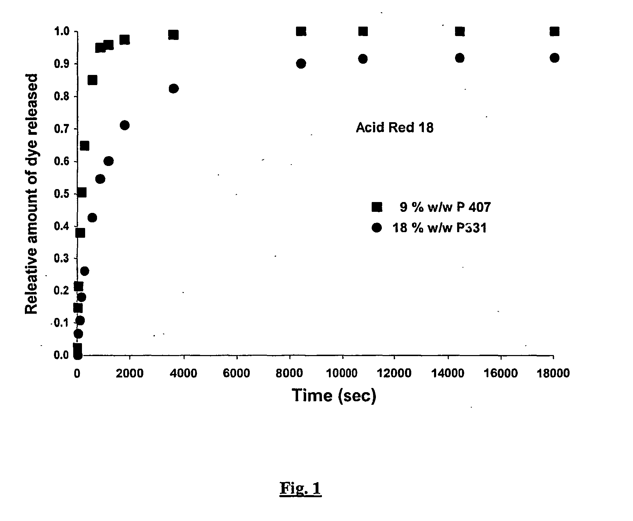

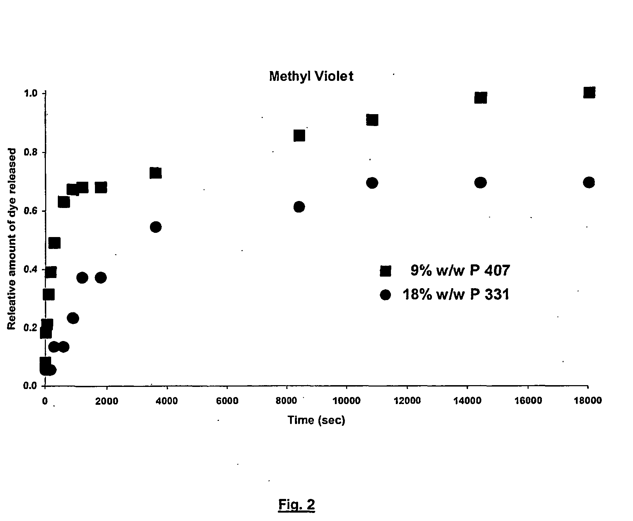

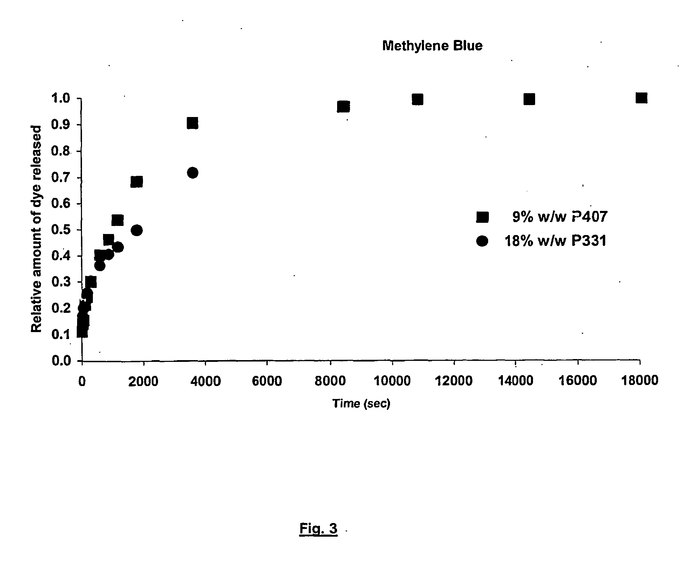

The results, shown in Figures 1- 4, are reported,in terms of a comparison of the relative amount of dyes released as a function of time from the various coating compositions, as the absolute release rates from such dressings will be dependent upon the specific contact conditions between the dressing and the wound under treatment; including contact surface area and volume of surrounding wound fluid. From Figures 1-3 it can be seen that at the same surface concentration of poloxamer, the rate of release of all three model dyes was significantly reduced from a coated layer of P331 compared to that from a coating layer of P407. Greater concentrations of P407 had no significant effect on release rates.

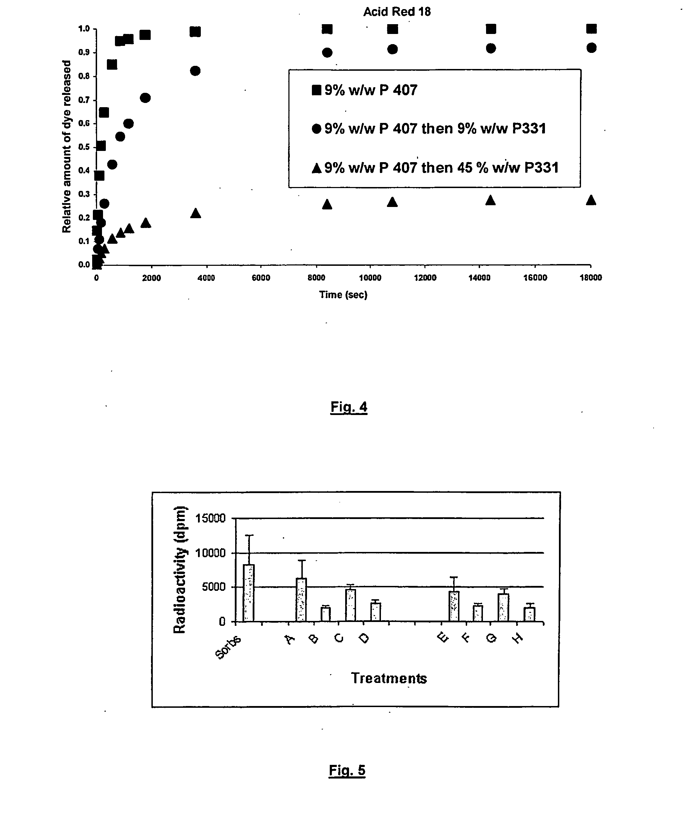

Figure 4 demonstrates the effects of depositing subsequent additional layers of P331 on the time dependent release of the model water soluble anionic dye, Acid Red 18. It can be seen that, using this multiple layer approach, it was possible to reduce the amount of dye released over a five hour period by approximately 80%, compared to the release characteristics from a single layer of P407 alone.

### EXAMPLE 5

### Adhesion Studies

This study establishes *an in vitro* model that can be used quantitatively to evaluate cell adhesion to wound dressings. Cells obtained from equine chronic wounds were used in this *in vitro* model to mimic (as closely as possible) the *in vivo* adhesive characteristics of cells involved in wound healing. Thus, the cells utilised were fibroblasts present in granulation tissue and epithelial cells responsible for re-epithelialisation. These cells are the most likely to come into direct contact with primary dressings during the wound healing process.

In this model, samples of an alginate dressing were treated with one of three different poloxamers with or without addition of 2% lidocaine. Both dressings were presoaked in a suitable fluid medium (e.g. either tissue culture media) in order to simulate a wound fluid environment. The dressings were applied to the surface of the cell cultures and allowed to `dry out' for a period of 24 h, in order to simulate the *in vivo* dehydration of a dressing on the wound surface. This time period allowed the outside of the dressing to become completely dry while the underside remained moist, this imitating the *in vivo* situation.

### Methods

### Fibroblast cell culture

Tissue was obtained *post mortem* from both the healing and non-healing areas of wounds in horses that had been killed for non-related clinical reasons. Tissue samples for fibroblast culture were treated as follows. Samples were immediately transferred to a dish, washed in Hank's balanced salt solution (HBSS, Gibco), cut into 3-5mm² pieces and placed into 25 cm² tissue culture flasks (Nunc, Gibco) containing Dulbecco's Modified Eagle Medium (DMEM, Gibco), supplemented with 10% foetal calf serum (FCS, Sigma). 20mM Hepes buffer, 100 µg/ml gentamicin and 0.5 µg/ml amphotericin B (Gibco). Incubation was at 37°C in a 5% CO₂/% air environment. Readiness for sub-culturing was determined by the extent of fibroblast cell outgrowth (5-10 days). Cells were farmed successively in a 1:4 split ratio to passage 3-8 times for experimental use. Fibroblasts were harvested from stock dishes and plated out at 2 x 10⁵ cells/dish onto either plastic or type I collagen (Sigma) at 2 mg/ml and the dishes were incubated as described above. Cells were left to attach for 24 h, after which the cells were metabolically labelled.

### Keratinocyte cell culture

Tissue was obtained as described above. Skin strips were rinsed 3 or 4 times in fresh DMEM (-FCS + supplements as above). The dermis was removed from the skin and the epithelium cut into small pieces about 3-4mm in size and subsequently digested in 0.1% collagenase at 37°C for 4 h. After incubation the solutions were collected and the tissue washed. The solution and washes were pooled and centrifuged to obtain cells. The cell pellet was rinsed 3 x with DMEM and plated into a large flask at 5 x 10⁵ cells per flask. Keratinocytes at passage 2 were harvested from stock dishes and plated out onto either plastic or type IV collagen coated dishes (Bio-coat, Becton Dickson) at 2 × 10⁵ cells/dish in 1 ml keratinocyte serum free medium (Gibco) supplemented with bovine pituitary extract (25 µg/ml) and epidermal growth factor (EGF) 0.2 ng/ml with 10% FCS and 100 µg/ml gentamicin. Cells were left to attach for 24 h after which the cells were metabolically labelled. Pure cultures of keratinocytes were obtained by eliminating fibroblasts by selective trypsinisation using 0.5% trypsin/EDTA for 5 min at 37°C.

### Metabolic labelling of cell cultures

The bioadhesion of cells was investigated using a tritiated thymidine assay. The cells were labelled with 2 µCi/100 µl [6-3 H]-thymidine in DMEM with 10% FCS for bioadhesion analysis and incubated. An equal amount of radiolabel was added to each culture and the cells were incubated for a further 24 h.

### Bioadhesion model and application of dressings

Media was removed from the cultures and the cell layer was washed with HBSS. The dressings used were a conventional, currently marketed viscose gauze wound contract dressing and a gelling dressing (MultiDRESS WCL - ConvaTec), and were cut into 1 cm² pieces. The dressings were soaked in fresh medium and separately placed on to the surface of the cell cultures using sterile forceps, with application of minimum force to maintain contact between the dressing and the culture. One dressing was added to each 35 mm cell culture dish, a total of 18 cultures were used for each separate analysis. Initially the dressings were left in place for different periods of time in order to evaluate the optimum time point that would simulate clinical conditions of the dressings 'drying out' on the wounds at a controlled temperature.

After the specified periods of time the dressings were removed in a consistent fashion by carefully peeling the dressing from the surface of the culture using sterile forceps. Minimum force was applied to avoid damaging the cells and causing any additional detachment of the cells from the dressing. The number of cells attached to the dressing was evaluated using quantitative (radio labelling and manual counting) and qualitative (photographic) techniques. Bovine serum albumin supplemented media was also used in the same series of assays at concentrations of 2.5, 5.0 and 1.0 mg/ml.

### Adhesion measurement techniques

Radio label technique: the dressings were removed from the cultures and placed into scintillation vials, scintillation fluid was added to each of the samples, thereafter the samples were counted in a liquid scintillation counter. The results were expressed as disintegrations per minute (dpm) x 100 for each of the dressings evaluated.

### Cell counting

The cell numbers in the triplicate parallel cultures were determined by trypsinisation of cell cultures before and after treatment then counted manually using a Neubauer counting chamber.

### Statistical analysis

All of the results were expressed as the mean ± standard deviation of the mean (SD). The statistical significance of the results was assessed with Students unpaired *t*-test, analysis of variance and Scheffe's multiple *t*-test (Statgraphics Software). A P value of <0.05 was considered to be significant.

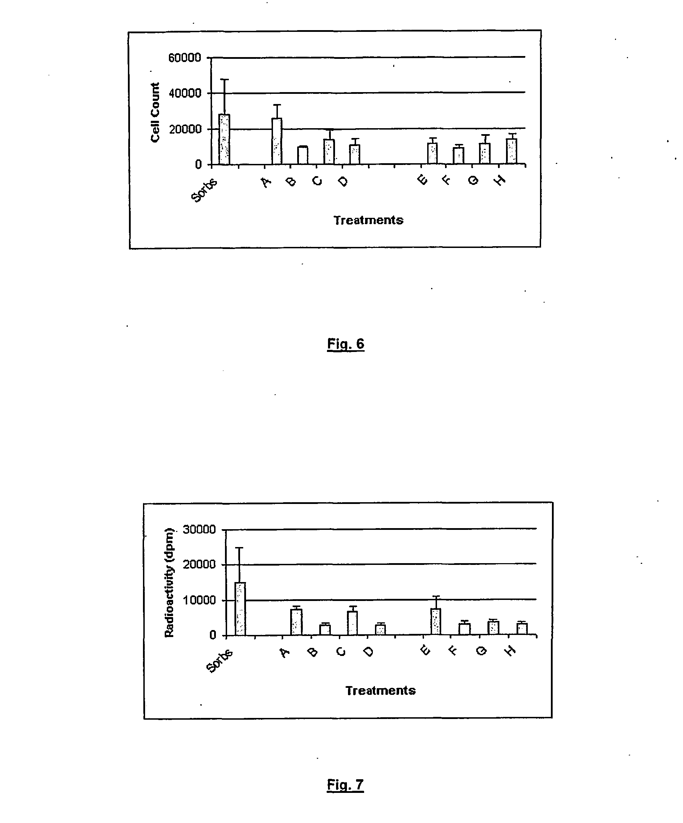

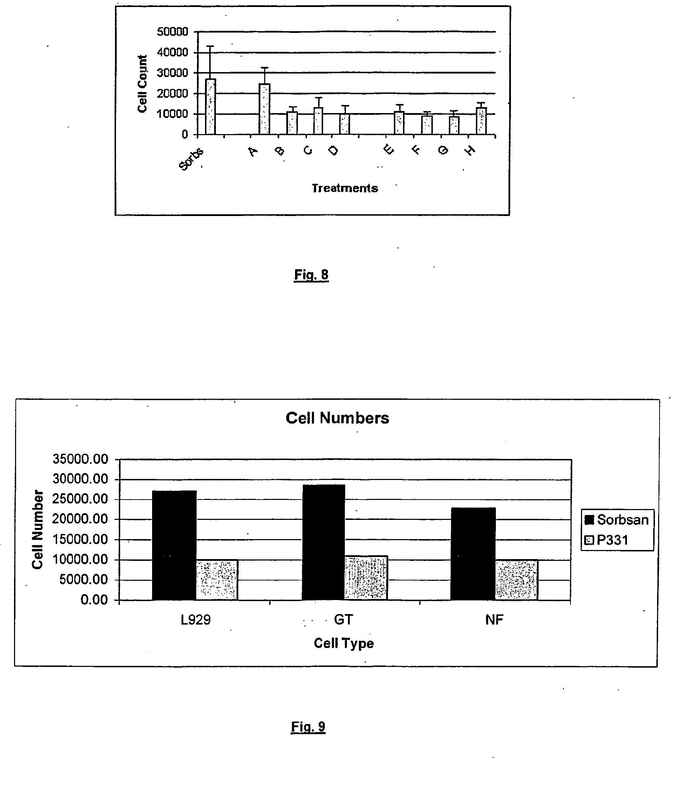

The results are shown in Figures 5 - 8 (granulation tissue cells - Figures 5 and 6, L929 cells Figures 7 and 8). In general, it can be seen that adhesion of the cells to the dressings followed a trend, in that the untreated Sorbsan (alginate dressing) showed the greatest level of adherence when compared to that of the poloxamer treated dressings.

### Key to Fig's:

- A: Poloxamer P234 ; Aqueous route of preparation; no Lidocaine
- B: Poloxamer P234 ; Aqueous route of preparation ; 0.2% Lidocaine
- C: Poloxamer P407 ; Aqueous route of preparation, no Lidocaine
- D: Poloxamer P407 ; Aqueous route of preparation 0.2% Lidocaine
- E: Poloxamer P234 ; Acetone route of preparation ; no Lidocaine
- F: Poloxamer P234 ; Acetone route of preparation; 0.2% Lidocaine
- G: Poloxamer P407 ; Acetone route of preparation ; no Lidocaine
- H: Poloxamer P407 ; Acetone route of preparation ; 0.2% Lidocaine

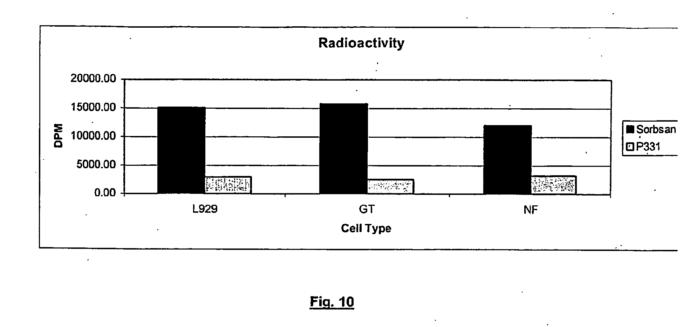

Figures 9 and 10 illustrate the effect of the presence of a surface layer of P331 on the adherence of various cell types to a fibrous alginate dressing. It can be seen that the presence of the poloxamer resulted in an approximate 70% reduction in the adherence of all cell types.

### Key to Figs 9 and 10

### Sorbsan - untreated fibrous alginate dressing

### P331 - Sorbsan dressing (Maersk medical) treated with 10% w/w P331 ; acetone route of preparation

### Cell types

GT - Granulation Tissue cells ; L 929 - Immortal cell line ; NF - normal fibroblasts

## Claims

1. An absorptive dressing for an exudarive wound, wherein at least that part of the dressing intended for contact with the wound has been coated with at least one poloxamer, and wherein the absorptive part is an alginate.

2. A dressing according to claim 1, wherein the dressing is highly absorbent

3. A dressing according to claim 1 or 2, which is not saturated with poloxamer.

4. A dressing according to claim 3, wherein the amount of poloxamer is between 0.5% and 200% by weight of the absorptive part of the dressing.

5. A dressing according to claim 4, wherein the amount of poloxamer is between I % and 50%,

6. A dressing according to any preceding claim, wherein the dressing is fibrous.

7. A dressing according to any preceding claim, having a backing which is impermeable to water and wound exudate.

8. A dressing according to any preceding claim, wherein the poloxamer is selected from one or more of P188, P234, P237, P331, P338 and P407.

9. A dressing according to any preceding claim, wherein the poloxamer has been applied thereto in at least two coats.

10. A dressing according to any preceding claim, wherein the poloxamer coating comprises at least two poloxamers.

11. A dressing according to claim 10, wherein the poloxamers are P407 and P331.

12. A dressing according to any preceding claim, wherein at least one additional therapeutically active substance is incorporated with the poloxamer.

13. A dressing according to claim 12, wherein the additional substance is selected from at least one of the group consisting of local anaesthetics, antibacterials, antifungals, anti-inflammatory agents, anti-cancer agents and wound treatment agents.

14. A dressing according to claim 13, comprising a local anaesthetic as an additional substance.

15. A dressing according to claim 14, wherein the local anaesthetic is lidocaine.

16. A dressing according to any preceding claim, comprising fibrous alginate treated with a poloxamer and a local anaesthetic, especially lidocaine.

17. A process for the preparation of a dressing according to any preceding claim, comprising exposing a suitable dressing, with or without a backing, to a poloxamer, or solution thereof.

18. A process according to claim 17, wherein the solution is aqueous or non-aqueous.

19. A process according to claim 18, wherein a solution of poloxamer in acetone is used.

20. A process according to any of claims 17 to 19, wherein the poloxamer, or solution thereof, comprises at least one additional therapeutically active substance.

21. A process according to claim 20, wherein the additional substance is lidocaine.

22. A process according to any of claims 17 to 21, which is repeated and wherein the poloxamer, or solution thereof, is the same or different for each time the process is performed.

23. A process according to claim 22, wherein at least one poloxamer or solution thereof comprises at least one additional therapeutically active substance.

24. A process according to any of claims 17 to 23, where a solution of poloxamer is used and is dried, or substantially dried, before use or before a repeat of the process.

## Patentansprüche

1. Saugfähiger Verband für eine exsudative Wunde, wobei zumindest der Teil des Verbands, der für den Kontakt mit der Wunde vorgesehen ist, mit mindestens einem Poloxamer beschichtet ist, und wobei der saugfähige Teil ein Alginat ist.

2. Verband nach Anspruch 1, wobei der Verband hochsaugfähig ist.

3. Verband nach Anspruch 1 oder 2, der nicht mit Poloxamer gesättigt ist.

4. Verband nach Anspruch 3, wobei der Poloxameranteil zwischen 0,5 und 200 Gew.-% des saugfähigen Teils des Verbands liegt.

5. Verband nach Anspruch 4, wobei der Poloxameranteil zwischen 1 Gew.-% und 50 Gew.-% liegt.

6. Verband nach einem der vorstehenden Ansprüche, wobei der Verband faserartig ist.

7. Verband nach einem der vorstehenden Ansprüche, der eine für Wasser und Wundexsudat undurchlässige Rückschicht aufweist.

8. Verband nach einem der vorstehenden Ansprüche, wobei das Poloxamer unter einem oder mehreren der Poloxamere P188, P234, P237, P331, P338 und P407 ausgewählt ist.

9. Verband nach einem der vorstehenden Ansprüche, wobei das Poloxamer in mindestens zwei Schichten darauf aufgebracht worden ist.

10. Verband nach einem der vorstehenden Ansprüche, wobei die Poloxamerbeschichtung mindestens zwei Poloxamere aufweist.

11. Verband nach Anspruch 10, wobei die Poloxamere P407 und P331 sind.

12. Verband nach einem der vorstehenden Ansprüche, wobei mindestens eine zusätzliche therapeutisch wirksame Substanz zusammen mit dem Poloxamer beigemischt wird.

13. Verband nach Anspruch 12, wobei die zusätzliche Substanz unter mindestens einem Element der Gruppe ausgewählt ist, die aus Lokalanästhetika, bakterienhemmenden Mitteln, Antimykotika, entzündungshemmenden Mitteln, Antikrebsmitteln und Wundbehandlungsmitteln besteht.

14. Verband nach Anspruch 13, der ein Lokalanästhetikum als zusätzliche Substanz aufweist.

15. Verband nach Anspruch 14, wobei das Lokalanästhetikum Lidokain ist.

16. Verband nach einem der vorstehenden Ansprüche, der faserförmiges Alginat aufweist, das mit einem Poloxamer und einem Lokalanästhetikum, speziell mit Lidokain, behandelt ist.

17. Verfahren zur Herstellung eines Verbands nach einem der vorstehenden Ansprüche mit Einwirkenlassen eines Poloxamers oder einer Lösung davon auf einen geeigneten Verband mit oder ohne Rückschicht.

18. Verfahren nach Anspruch 17, wobei die Lösung wäßrig oder nichtwäßrig ist.

19. Verfahren nach Anspruch 18, wobei eine Lösung von Poloxamer in Aceton verwendet wird.

20. Verfahren nach einem der Ansprüche 17 bis 19, wobei das Poloxamer oder dessen Lösung mindestens eine zusätzliche therapeutisch wirksame Substanz aufweist.

21. Verfahren nach Anspruch 20, wobei die zusätzliche Substanz Lidokain ist.

22. Verfahren nach einem der Ansprüche 17 bis 21, das wiederholt wird, wobei das Poloxamer oder dessen Lösung für jede Wiederholung des Verfahrens gleich oder unterschiedlich ist.

23. Verfahren nach Anspruch 22, wobei mindestens ein Poloxamer oder dessen Lösung mindestens eine zusätzliche therapeutisch wirksame Substanz aufweist.

24. Verfahren nach einem der Ansprüche 17 bis 23, wobei eine Poloxamerlösung verwendet und vor dem Gebrauch oder vor einer Wiederholung des Verfahrens getrocknet oder weitgehend getrocknet wird.

## Revendications

1. Pansement absorbant pour une plaie exsudative, dans lequel au moins la partie du pansement prévue pour être en contact avec la plaie a été recouverte avec au moins un poloxamère, et dans lequel la partie absorbante est un alginate.

2. Pansement selon la revendication 1, dans lequel le pansement est hautement absorbant.

3. Pansement selon la revendication 1 ou 2, qui n'est pas saturé avec du poloxamère.

4. Pansement selon la revendication 3, dans lequel la quantité de poloxamère est comprise entre 0,5% et 200% par poids de la partie absorbante du pansement.

5. Pansement selon la revendication 4, dans lequel la quantité de poloxamère est comprise entre 1 % et 50%.

6. Pansement selon l'une quelconque des revendications précédentes, dans lequel le pansement est fibreux.

7. Pansement selon l'une quelconque des revendications précédentes, ayant un support qui est imperméable à l'eau et à l'exsudat de la plaie.

8. Pansement selon l'une quelconque des revendications précédentes, dans lequel le poloxamère est choisi parmi un ou plusieurs de P188, P234, P237, P331, P338 et P407.

9. Pansement selon l'une quelconque des revendications précédentes, dans lequel le poloxamère a été appliqué sur celui-ci en au moins deux couches.

10. Pansement selon l'une quelconque des revendications précédentes, dans lequel le revêtement de poloxamère comprend au moins deux poloxamères.

11. Pansement selon la revendication 10, dans lequel les poloxamères sont du P407 et du P331.

12. Pansement selon l'une quelconque des revendications précédentes, dans lequel au moins une substance thérapeutiquement active additionnelle est incorporée avec le poloxamère.

13. Pansement selon la revendication 12, dans lequel la substance additionnelle est choisie parmi au moins un du groupe qui comprend des anesthésiques locaux, des antibactériens, des antifongiques, des agents anti-inflammatoires, des agents anti-cancer et des agents de traitement de plaie.

14. Pansement selon la revendication 13, comprenant un anesthésique local comme une substance additionnelle.

15. Pansement selon la revendication 14, dans lequel l'anesthésique local est de la lidocaïne.

16. Pansement selon l'une quelconque des revendications précédentes, comprenant de l'alginate fibreux traité avec un poloxamère et un anesthésique local, particulièrement de la lidocaïne.

17. Procédé pour la préparation d'un pansement selon l'une quelconque des revendications précédentes, comprenant les étapes d'exposer un pansement adéquat, avec ou sans support, à un poloxamère, ou à une solution contenant celui-ci.

18. Procédé selon la revendication 17, dans lequel la solution est aqueuse ou non aqueuse.

19. Procédé selon la revendication 18, dans lequel une solution contenant du poloxamère dans de l'acétone est utilisée.

20. Procédé selon l'une quelconque des revendications 17 à 19, dans lequel le poloxamère, ou la solution contenant celui-ci, comprend au moins une substance thérapeutiquement active additionnelle.

21. Procédé selon la revendication 20, dans lequel la substance additionnelle est de la lidocaïne.

22. Procédé selon l'une quelconque des revendications 17 à 21, qui est répété et dans lequel le poloxamère, ou la solution contenant celui-ci, est le même ou est différent à chaque répétition du procédé.

23. Procédé selon la revendication 22, dans lequel au moins un poloxamère ou une solution contenant celui-ci comprend au moins une substance thérapeutiquement active additionnelle.

24. Procédé selon l'une quelconque des revendications 17 à 23, dans lequel une solution contenant du poloxamère est utilisée et est séchée, ou substantiellement séchée, avant usage ou avant une répétition du procédé.
